# EUROPEAN PATENT SPECIFICATION

(11) **EP 1 055 665 B1**
(45) Date of publication and mention of the grant of the patent: **22.10.2003**
(21) Application number: 00304372.6
(22) Date of filing: 24.05.2000
(51) Int. Cl.: C07C 237/20, C07C 237/24, C07D 277/30, A61K 31/165, A61P 29/00

(54) **3-(3-Hydroxyphenyl)-3-amino-propionamide derivatives**
3-(3-Hydroxyphenyl)-3-amino-propionamidderivate
Dérivés de la 3-(3-hydroxyphényl)-3-amino-propionamide

(30) Priority: 28.05.1999 US 136548 P
(43) Date of publication of application: 29.11.2000
(73) Proprietor: Pfizer Products Inc., Groton, Connecticut 06340 (US)
(72) Inventor: Liras, Spiros, Groton, Connecticut 06340 (US)
(74) Representative: Simpson, Alison Elizabeth Fraser

(56) References cited:
- WO-A-96/22276
- US-A- 5 364 850
- US-A- 5 389 645

## Description

This invention relates to 3-(3-hydroxyphenyl)-3-amino-propionamide derivatives which have utility as ligands for opioid receptors.

In the study of opioid biochemistry, a variety of endogenous opioid compounds and non-endogenous opioid compounds has been identified. In this effort, significant research has been focused on understanding the mechanism of opioid drug action, particlarly as it relates to cellular and differentiated tissue opioid receptors.

Opioid drugs are typically classified by their binding selectivity in respect of the cellular and differentiated tissue receptors to which a specific drug species binds as a ligand. These receptors include mu (µ), delta (δ) and kappa (κ) receptors.

At least three subtypes of opioid receptors (mu, delta and kappa) are described and documented in the scientific literature. All three receptors are present in the central and peripheral nervous systems of many species including man. Activation of delta receptors produces antinociception in rodents and can induce analgesia in man, in addition to influencing motility of the gastrointestinal tract. (See Burks, T.F. (1995) in "The Pharmacology of Opioid Peptides", edited by Tseng, L.F., Harwood Academic Publishers).

International Patent Publication No. WO96/22276 discloses ethylamine derivatives. US Patent No. 5 389 645 discloses substituted tyrosyl diamide compounds which are useful for inducing analgesia in animals. US Patent No. 5 364 850 also discloses substituted tyrosyl diamide compounds which are useful for inducing analgesia in animals.

The well known narcotic opiates such as morphine and its analogs are selective for the opioid mu receptor. Mu receptors mediate analgesia, respiratory depression, and inhibition of gastrointestinal transit. Kappa receptors mediate analgesia and sedation.

The existence of the opioid delta receptor is a relatively recent discovery which followed the isolation and characterization of endogenous enkephalin peptides, which are ligands for the delta receptor. Research in the past decade has produced significant information about the delta receptor, but a clear picture of its function has not yet emerged. Delta receptors mediate analgesia, but do not appear to inhibit intestinal transit in the manner characteristic of mu receptors.

There is a continuing need in the art for improved opioid compounds, particularly compounds which are free of addictive character and other adverse side effects of conventional opiates such as morphine and pethidine.

The present inventor has discovered a novel class of 3-(3-hydroxyphenyl)-3-amino-propionamide derivatives that are potent and selective mu opioid ligands and are useful for treatment of rejection in organ transplants and skin grafts, epilepsy, chronic pain, neurogenic pain, nonsomatic pain, stroke, cerebral ischemica, shock, head trauma, spinal cord trauma, brain edema, Hodgkin's disease, Sjogren's disease, systemic lupus erythematosis, analgesia, addiction, immunotherapy, appetite control, gastrointestinal dysfunction, gastrointestinal disorders such as gastritis, functional bowel disease, irritable bowel syndrome, functional diarrhoea, functional distention, nonulcerogenic dyspepsia and other disorders of motility or secretion, and emesis, acute pain, chronic pain, neurogenic pain, nonsomatic pain, allergies, respiratory disorders such as asthma, cough and apnea, inflammatory disorders such as rheumatoid arthritis, osteoarthristis, psoriasis and inflammatory bowel disease, urogenital tract disorders such as urinary incontinence, hypoxia (e.g., perinatal hypoxia), hypoglycemic neuronal damage, chemical dependencies and addictions (e.g., a dependency on, or addiction to opiates, benzodiazepines, cocaine, nicotine or ethanol), drug or alcohol withdrawal symptoms, and cerebral deficits subsequent to cardiac bypass surgery and grafting.

### Summary of the Invention

This invention relates to a compound of the formula 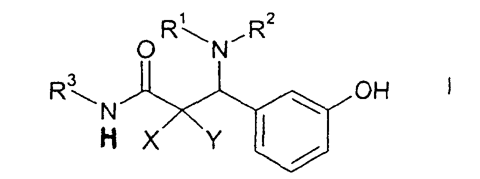 or the pharmaceutically acceptable salt thereof; wherein
R¹, R², and R³ are each independently selected from hydrogen, (C₁-C₆)alkyl, (C₆-C₁₀)aryl, (C₆-C₁₀)aryl(C₁-C₆)alkyl, (C₂-C₉)heteroaryl, (C₂-C₉)heteroaryl(C₁-C₆)alkyl, (C₃-C₇)cycloalkyl, (C₃-C₇)cycloalkyl(C₁-C₆)alkyl, (C₂-C₉)heterocycloalkyl and (C₂-C₉)heterocycloalkyl(C₁-C₆)alkyl wherein each alkyl, aryl, heteroaryl, cycloalkyl or heterocycloalkyl may be optionally substituted by one to four substituents selected from the group consisting of carboxy, cyano, amino, deuterium, hydroxy, (C₁-C₆)alkyl, (C₁-C₆)alkoxy, halo, (C₁-C₆)acyl, (C₁-C₆)alkylamino, amino(C₁-C₆)alkyl, (C₁-C₆)alkoxy-CO-NH, (C₁-C₆)alkylamino-CO-, (C₂-C₆)alkenyl, (C₂-C₆) alkynyl, (C₁-C₆)alkylamino, amino(C₁-C₆)alkyl, hydroxy(C₁-C₆)alkyl, (C₁-C₆)alkoxy(C₁-C₆)alkyl, (C₁-C₆)acyloxy(C₁-C₆)alkyl, nitro, cyano(C₁-C₆)alkyl, halo(C₁-C₆)alkyl, nitro(C₁-C₆)alkyl, trifluoromethyl, trifluoromethyl(C₁-C₆)alkyl, (C₁-C₆)acylamino, (C₁-C₆)acylamino(C₁-C₆)alkyl, (C₁-C₆)alkoxy(C₁-C₆)acylamino, amino(C₁-C₆)acyl, amino(C₁-C₆)acyl(C₁-C₆)alkyl, (C₁-C₆)alkylamino(C₁-C₆)acyl, ((C₁-C₆)alkyl)₂amino(C₁-C₆)acyl, R⁴R⁵N-CO-O-, R⁴R⁵N-CO-(C₁-C₆)alkyl, (C₁-C₆)alkyl-S(O)ₘ, R⁴R⁵NS(O)ₘ, R⁴R⁵NS(O)ₘ (C₁-C₆)alkyl, R⁴S(O)ₘ R⁵N, R⁴S(O)ₘR⁵N(C₁-C₆)alkyl wherein m is 0, 1 or 2 and R⁴ and R⁵ are each independently selected from hydrogen or (C₁-C₆)alkyl; and
X and Y are each independently hydrogen or (C₁₋C₃)alkyl;
or X and Y may be taken together with the carbon to which they are attached to form a compound of formula II 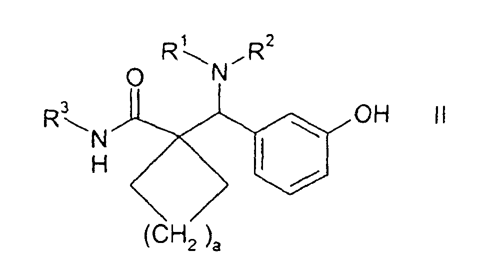 wherein
a is 0, 1, 2, 3, or 4; and
R¹, R² and R³ are as defined above.

The term "alkyl", as used herein, unless otherwise indicated, includes saturated monovalent hydrocarbon radicals having straight, branched or cyclic moieties or combinations thereof.

The term "alkoxy", as used herein, includes O-alkyl groups wherein "alkyl" is defined above.

The term "halo", as used herein, unless otherwise indicated, includes fluoro, chloro, bromo or iodo.

The compounds of this invention may contain double bonds. When such bonds are present, the compounds of the invention exist as cis and trans configurations and as mixtures thereof.

Unless otherwise indicated, the alkyl and alkenyl groups referred to herein, as well as the alkyl moieties of other groups referred to herein (e.g., alkoxy), may be linear or branched, and they may also be cyclic (e.g., cyclopropyl, cyclobutyl, cyclopentyl, cyclohexyl or cycloheptyl) or be linear or branched and contain cyclic moieties. Unless otherwise indicated, halogen includes fluorine, chlorine, bromine, and iodine.

(C₃-C₁₀)Cycloalkyl when used herein refers to cycloalkyl groups containing zero to two levels of unsaturation such as cyclopropyl, cyclobutyl, cyclopentyl, cyclopentenyl, cyclohexyl, cyclohexenyl, 1,3-cyclohexadiene, cycloheptyl, cycloheptenyl, bicyclo[3.2.1]octane, norbornanyl etc..

(C₂-C₉)Heterocycloalkyl when used herein refers to pyrrolidinyl, tetrahydrofuranyl, dihydrofuranyl, tetrahydropyranyl, pyranyl, thiopyranyl, aziridinyl, oxiranyl, methylenedioxyl, chromenyl, isoxazolidinyl, 1,3-oxazolidin-3-yl, isothiazolidinyl, 1,3-thiazolidin-3-yl, 1,2-pyrazolidin-2-yl, 1,3-pyrazolidin-1-yl, piperidinyl, thiomorpholinyl, 1,2-tetrahydrothiazin-2-yl, 1,3-tetrahydrothiazin-3-yl, tetrahydrothiadiazinyl, morpholinyl, 1,2-tetrahydrodiazin-2-yl, 1,3-tetrahydrodiazin-1-yl, tetrahydroazepinyl, piperazinyl, chromanyl, etc. One of ordinary skill in the art will understand that the connection of said (C₂-C₉)heterocycloalkyl rings is through a carbon or a sp³ hybridized nitrogen heteroatom.

(C₂-C₉)Heteroaryl when used herein refers to furyl, thienyl, thiazolyl, pyrazolyl, isothiazolyl, oxazolyl, isoxazolyl, pyrrolyl, triazolyl, tetrazolyl, imidazolyl, 1,3,5-oxadiazolyl, 1,2,4-oxadiazolyl, 1,2,3-oxadiazolyl, 1,3,5-thiadiazolyl, 1,2,3-thiadiazolyl, 1,2,4-thiadiazolyl, pyridyl, pyrimidyl, pyrazinyl, pyridazinyl, 1,2,4-triazinyl, 1,2,3-triazinyl, 1,3,5-triazinyl, pyrazolo[3,4-b]pyridinyl, cinnolinyl, pteridinyl, purinyl, 6,7-dihydro-5H-[1]pyrindinyl, benzo[b]thiophenyl, 5, 6, 7, 8-tetrahydro-quinolin-3-yl, benzoxazolyl, benzothiazolyl, benzisothiazolyl, benzisoxazolyl, benzimidazolyl, thianaphthenyl, isothianaphthenyl, benzofuranyl, isobenzofuranyl, isoindolyl, indolyl, indolizinyl, indazolyl, isoquinolyl, quinolyl, phthalazinyl, quinoxalinyl, quinazolinyl, benzoxazinyl; etc. One of ordinary skill in the art will understand that the connection of said (C₂-C₉)heterocycloalkyl rings is through a carbon atom or a sp³ hybridized nitrogen heteroatom.

(C₆-C₁₀)aryl when used herein refers to phenyl or naphthyl.

Preferred compounds of formula I include those wherein X is hydrogen, (C₁-C₆)alkyl or (C₃-C₇)cycloalkyl.

Other preferred compounds of formula I include those wherein Y is hydrogen, (C₁-C₆)alkyl or (C₃-C₇)cycloalkyl.

Other preferred compounds of formula I include those wherein R¹ is hydrogen or (C₁-C₆)alkyl.

Other preferred compounds of formula I include those wherein R² is (C₁-C₆)alkyl, (C₃-C₇)cycloalkyl(C₁-C₆)alkyl or trifluoromethyl.

Other preferred compounds of formula I include those wherein R³ is (C₃-C₇)cycloalkyl(C₁-C₇)alkyl or (C₆-C₁₀)aryl(C₁-C₆)alkyl.

More preferred compounds of formula I include those wherein X is hydrogen, (C₁-C₆)alkyl or (C₃-C₇)cycloalkyl; Y is hydrogen, (C₁-C₆)alkyl or (C₃-C₇)cycloalkyl; R¹ is hydrogen or (C₁-C₆)alkyl; R² is (C₁-C₆)alkyl, (C₃-C₇)cycloalkyl(C₁-C₆)alkyl or trifluoromethyl; and R³ is (C₃-C₇)cycloalkyl(C₁-C₇)alkyl or (C₆-C₁₀)aryl(C₁-C₆)alkyl.

The compounds of formula I and their pharmaceutically acceptable salts are mu opioid receptor ligands and are useful in the treatment of a variety of neurological and gastrointestinal disorders. Examples of disorders that can be treated with the compounds of formula I and their pharmaceutically acceptable salts are rejection in organ transplants and skin grafts, epilepsy, chronic pain, neurogenic pain, nonsomatic pain, stroke, cerebral ischemica, shock, head trauma, spinal cord trauma, brain edema, Hodgkin's disease, Sjogren's disease, systemic lupus erythematosis, analgesia, addiction, immunotherapy, appetite control, gastrointestinal dysfunction, gastrointestinal disorders such as gastritis, functional bowel disease, irritable bowel syndrome, functional diarrhoea, functional distention, nonulcerogenic dyspepsia and other disorders of motility or secretion, and emesis, acute pain, chronic pain, neurogenic pain, nonsomatic pain, allergies, respiratory disorders such as asthma, cough and apnea, inflammatory disorders such as rheumatoid arthritis, osteoarthritis, psoriasis and inflammatory bowel disease, urogenital tract disorders such as urinary incontinence, hypoxia (e.g., perinatal hypoxia), hypoglycemic neuronal damage, chemical dependencies and addictions (e.g., a dependency on, or addiction to opiates, benzodiazepines, cocaine, nicotine or ethanol), drug or alcohol withdrawal symptoms, and cerebral deficits subsequent to cardiac bypass surgery and grafting.

The present invention also relates to the pharmaceutically acceptable acid addition and base addition salts of compounds of the formula I. The acids which are used to prepare the pharmaceutically acceptable acid addition salts of the aforementioned base compounds of this invention are those which form non-toxic acid addition salts, i.e., salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [i.e., 1,1'-methylene-bis-(2-hydroxy-3-naphthoate)]salts. The chemical bases that are used as reagents to prepare the pharmaceutically acceptable base salts of this invention are those which form non-toxic base salts with the acidic compounds of formula I. Such non-toxic base salts include those derived from such pharmacologically acceptable cations as sodium, potassium, calcium and magnesium, etc.

The present invention also relates to the pharmaceutically acceptable base addition salts of compounds of the formula I. These salts are all prepared by conventional techniques. The chemical bases that are used as reagents to prepare the pharmaceutically acceptable base salts of this invention are those which form non-toxic base salts with the acidic compounds of formula I. Such non-toxic base salts include those derived from such pharmacologically acceptable cations as sodium, potassium, calcium and magnesium, etc.

For a review on pharmaceutically acceptable salts, see Berge et al., J. Pharm. Sci., 66, 1-19(1977).

This invention also relates to a pharmaceutical composition for treating a disorder or condition, the treatment or prevention of which can be effected or facilitated by modulating (i.e., increasing or decreasing) binding to mu opioid receptors in a mammal, including a human, comprising an amount of a compound of the formula I, or a pharmaceutically effective salt thereof, that is effective in treating such disorder or condition and a pharmaceutically acceptable carrier.

This invention also relates to the use of the present compounds for the preparation of a medicament for treating a disorder or condition, the treatment of which can be effected or facilitated by modulating binding to mu opioid receptors in a mammal.

This invention also relates to a pharmaceutical composition for treating a disorder or condition selected from inflammatory diseases such as arthritis (e.g., rheumatoid arthritis and osteoarthritis), psoriasis, asthma, or inflammatory bowel disease, disorders of respiratory function such as asthma, cough and apnea, allergies, gastrointestinal disorders such as gastritis, functional bowel disease, irritable bowel syndrome, functional diarrhoea, functional distension, functional pain, nonulcerogenic dyspepsia and other disorders of motility or secretion, and emesis, stroke, shock, brain edema, head trauma, spinal cord trauma, cerebral ischemia, cerebral deficits subsequent to cardiac bypass surgery and grafting, urogential tract disorders such as urinary incontinence, chemical dependencies and addictions (e.g., addictions to or dependencies on alcohol, opiates, benzodiazepines, nicotine, heroin or cocaine), chronic pain, nonsomatic pain, acute pain and neurogenic pain, systemic lupus erythematosis, analgesia, addiction, immunotherapy, appetite control, gastrointestinal dysfunction, Hodgkin's disease, Sjogren's disease, epilepsy and rejection in organ transplants and skin grafts in a mammal, including a human, comprising a glutamate neurotransmission modulating effective amount of a compound of the formula I, or a pharmaceutically salt thereof, and a pharmaceutically acceptable carrier.

This invention also relates to the use of the present compounds for the preparation of a medicament for treating a condition selected from inflammatory diseases such as arthritis, psoriasis, asthma, or inflammatory bowel disease, disorders of respiratory function such as asthma, cough and apnea, allergies, gastrointestinal disorders such as gastritis, functional bowel disease, irritable bowel syndrome, functional diarrhoea, functional distension, functional pain, nonulcerogenic dyspepsia and other disorders of motility or secretion, and emesis, stroke, shock, brain edema, head trauma, spinal cord trauma, cerebral ischemia, cerebral deficits subsequent to cardiac bypass surgery and grafting, urogential tract disorders such as urinary incontinence, chemical dependencies and addictions (e.g., addictions to or dependencies on alcohol, opiates, benzodiazepines, nicotine, heroin or cocaine), chronic pain, nonsomatic pain, acute pain and neurogenic pain, systemic lupus erythematosis, analgesia, addiction, immunotherapy, appetite control, gastrointestinal dysfunction, Hodgkin's disease, Sjogren's disease, epilepsy and rejection in organ transplants and skin grafts, in a mammal.

This invention also relates to a pharmaceutical composition for treating a disorder or condition, the treatment of which can be effected or facilitated by modulating binding to mu opioid receptors in a mammal, including a human, comprising an opioid receptor binding modulating effective amount of a compound of the formula I, or a pharmaceutically acceptable salt thereof, and a pharmaceutically acceptable carrier.

This invention also relates to the use of the present compounds for the preparation of a medicament for treating a disorder or condition, the treatment of which can be effected or facilitated by modulating in a mammal, including a human, binding to an mu opioid receptor.

This invention also relates to the use of the present compounds for the preparation of a medicament for treating a condition selected from inflammatory diseases such as arthritis, psoriasis, asthma, or inflammatory bowel disease, disorders of respiratory function such as asthma, cough and apnea, allergies, gastrointestinal disorders such as gastritis, functional bowel disease, irritable bowel syndrome, functional diarrhoea, functional distension, functional pain, nonulcerogenic dyspepsia and other disorders of motility or secretion, and emesis, stroke, shock, brain edema, head trauma, spinal cord trauma, cerebral ischemia, cerebral deficits subsequent to cardiac bypass surgery and grafting, urogential tract disorders such as urinary incontinence, chemical dependencies and addictions (e.g., addictions to or dependencies on alcohol, opiates, benzodiazepines, nicotine, heroin or cocaine), chronic pain, nonsomatic pain, acute pain and neurogenic pain, systemic lupus erythematosis, analgesia, addiction, immunotherapy, appetite control, gastrointestinal dysfunction, Hodgkin's disease, Sjogren's disease, epilepsy and rejection in organ transplants and skin grafts in a mammal.

This invention also relates to a pharmaceutical composition for treating a condition selected from inflammatory diseases such as arthritis, psoriasis, asthma, or inflammatory bowel disease, disorders of respiratory function such as asthma, cough and apnea, allergies, gastrointestinal disorders such as gastritis, functional bowel disease, irritable bowel syndrome, functional diarrhoea, functional distension, functional pain, nonulcerogenic dyspepsia and other disorders of motility or secretion, and emesis, stroke, shock, brain edema, head trauma, spinal cord trauma, cerebral ischemia, cerebral deficits subsequent to cardiac bypass surgery and grafting, urogential tract disorders such as urinary incontinence, chemical dependencies and addictions (e.g., addictions to or dependencies on alcohol, opiates, benzodiazepines, nicotine, heroin or cocaine), chronic pain, nonsomatic pain, acute pain and neurogenic pain, systemic lupus erythematosis, analgesia, addiction, immunotherapy, appetite control, gastrointestinal dysfunction, Hodgkin's disease, Sjogren's disease, epilepsy and rejection in organ transplants and skin grafts in a mammal, comprising an amount of a compound of the formula I that is effective in treating such condition and a pharmaceutically acceptable carrier.

### Detailed Description

The following reaction Scheme illustrates the preparation of the compounds of the present invention. Unless otherwise indicated X, Y. R¹, R² and R³ in the reaction Scheme and the discussion that follow are defined as above.

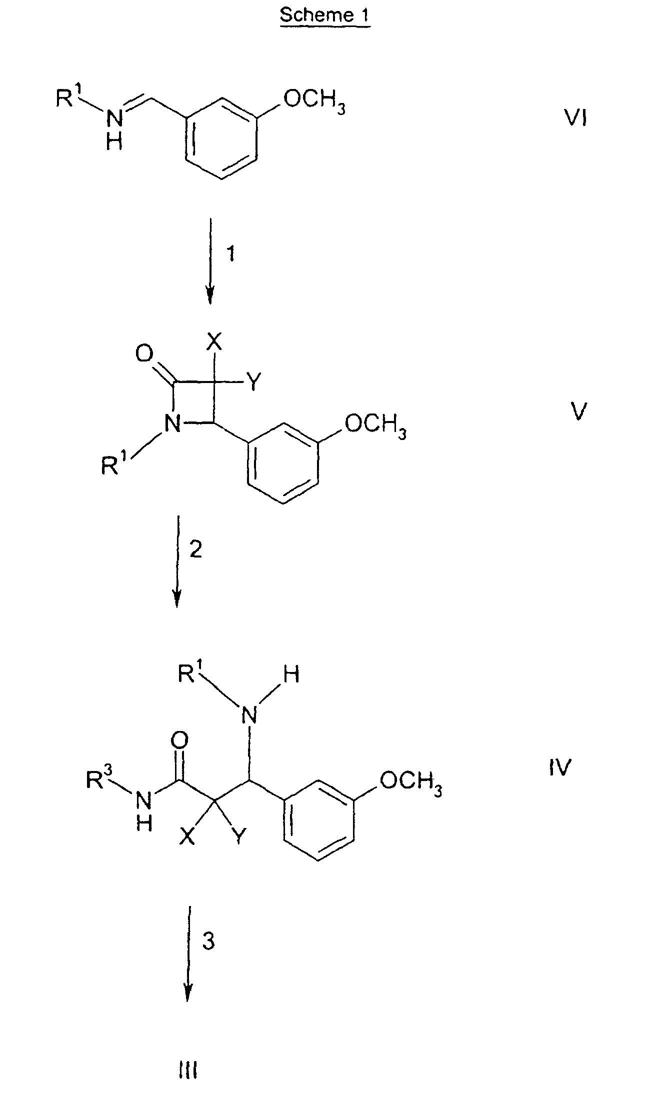

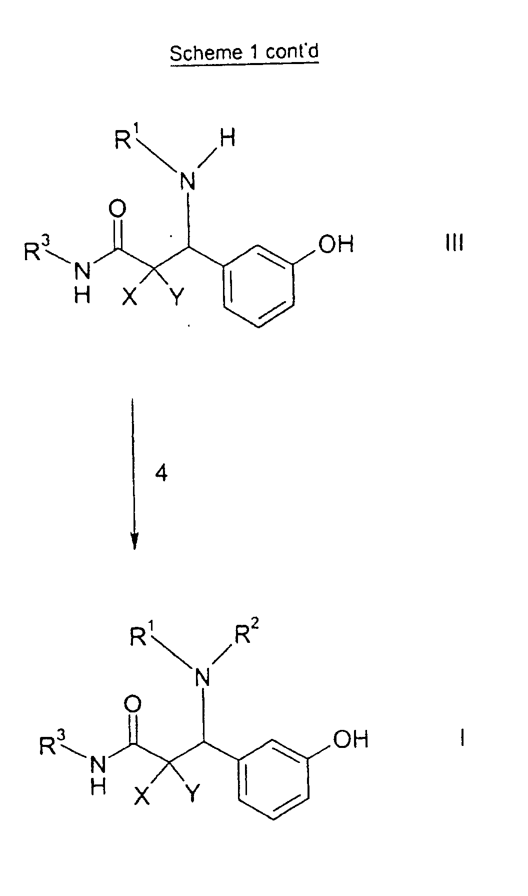

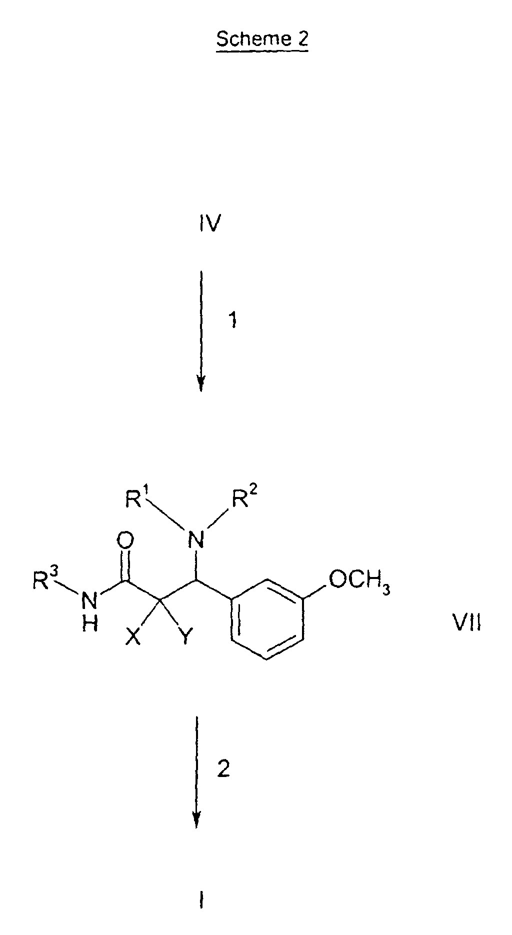

In reaction 1 of Scheme 1, the imine compound of formula **VI** is converted to the corresponding lactam compound of formula **V** by reacting **VI** with a ketene acetal of the formula 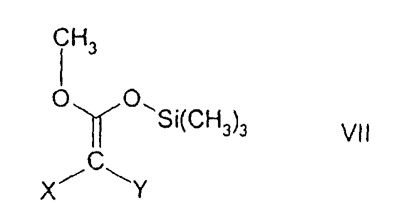 in the presence of an acid and aprotic solvent. Suitable acids include boron trifluoride diethyl etherate and titanium tetrachloride, and suitable aprotic solvents include methylene chloride, 1,2-dichloroethane and toluene. The reaction is conducted at a temperature between about -78°C to the reflux temperature of the solvent, preferably room temperature, for a time period between about 2 hours to about 24 hours, preferably about 12 hours.

In reaction 2 of Scheme 1, the lactam compound of formula **V** is converted to the corresponding amide compound of formula **IV** by reacting **V** with lithium amides of primary amines in the presence of an aprotic solvent, such as tetrahydrofuran and diethyl ether. The reaction is carried out at a temperature between about -78°C to the reflux temperature of the solvent, preferably about -78°C, for a time period between about 1 hours to about 4 hours, preferably about 2 hours.

In reaction 3 of Scheme 1, the amide compound of formula **IV** is converted to the corresponding compound of formula **III** by treating **IV** with boron tribromide in a polar aprotic solvent, such as methylene chloride, at a temperature between about -78°C to room temperature, preferably about 24°C. The reaction mixture is stirred for a time period between about 1 hours to about 16 hours, preferably about 4 hours. Alternatively, the compound of formula **III** is prepared by heating to reflux a mixture of **IV**, acetic acid and 40% aqueous hydrobromic acid, for a time period between about 2 hours to about 8 hours, preferably about 6 hours.

In reaction **4** of Scheme 1, the compound of formula **III** is converted to the corresponding compound of formula **I** by reacting **III** with an aldehyde of the formula, R²C(O)H, wherein R² is (C₁-C₆)alkyl, (C₆-C₁₀)aryl(C₁-C₆)alkyl, (C₂-C₉)heteroaryl(C₁-C₆)alkyl, (C₃-C₇)cycloalkyl(C₁-C₆)alkyl or (C₂-C₉)heterocycloalkyl(C₁-C₆)alkyl, in the presence of sodium triacetoxyborohydride in methylene chloride or sodium cyanoborohydride in methanol. The reaction is carried out at room temperature for a time period between about 2 hours to about 24 hours, preferably about 16 hours.

In reaction 1 of Scheme 2, the compound of formula **IV** is converted to the corresponding compound of formula **VII** by reacting **IV** with an Ikylating or arylating agent of the formula, R²X, wherein R² is primary or secondary (C₁-C₆)alkyl, (C₂-C₉)heteroaryl, (C₃-C₇)cycloalkylor (C₂-C₉)heterocycloalkyl, and X is a leaving group such as chloro, bromo, iodo, triflate, mesylate, tosylate, sodium or potassium carbonate or other alkali metal carbonate or bicarbonate bases. The reaction is carried out in solvent, such as dimethyl formamide or 1,2-dichloroethane, at a temperature between about 20°C to about 100°C, preferably about 80°C, for a time period between about 2 hours to about 48 hours, preferably about 24 hours. Alternatively, the compound of formula **VII** may be formed by reacting **IV** with a compound of the formula, R²X, wherein R² is optionally substituted (C₆-C₁₀)aryl or optionally substituted (C₂-C₉)heteroaryl and X is bromo, in the presence of a base, such as cesium carbonate or other alkali metal carbonates, a palladium catalyst, such as palladium acetate or palladium dibenzylideneacetone, and a suitable ligand, such as 2,2'-bis(diphenylphosphino)-1,1'-binaphthyl. The reaction is carried out in solvent, such as toluene or 1,4-dioxane, at a temperature between about 20°C to about 100°C, preferably about 90°C, for a time period between about 4 hours to about 24 hours, preferably about 16 hours.

In reaction 2 of Scheme 2, the compound of formula **VII** is converted to the corresponding compound of formula I by treating **VII** with boron tribromide in a polar aprotic solvent, such as methylene chloride, at a temperature between about -78°C to room temperature, preferably about 24°C. The reaction mixture is stirred for a time period between about 1 hours to about 16 hours, preferably about 4 hours. Alternatively, the compound of formula **III** is prepared by heating to reflux a mixture of **IV,** acetic acid and 40% aqueous hydrobromic acid, for a time period between about 2 hours to about 8 hours, preferably about 6 hours.

The starting materials used in the processes of Schemes 1 and 2 are either commercially available, known in the literature, or readily obtainable from commercially available or known compounds using methods that are well known in the art or described above.

Unless indicated otherwise, the pressure of each of the above reactions is not critical. Generally, the reactions will be conducted at a pressure from about one to about three atmospheres, preferably at ambient pressure (about one atmosphere).

The preparation of other compounds of the formula I not specifically described in the foregoing experimental section can be accomplished using combinations of the reactions described above that will be apparent to those skilled in the art.

The compounds of the formula I that are basic in nature are capable of forming a wide variety of different salts with various inorganic and organic acids. The acid that can be used to prepare the pharmaceutically acceptable acid addition salts of the base compounds of this invention are those which form non-toxic acid addition salts, i.e., salts containing pharmacologically acceptable anions, such as hydrochloride, hydrobromide, hydroiodide, nitrate, sulfate or bisulfate, phosphate or acid phosphate, acetate, lactate, citrate or acid citrate, tartrate or bitartrate, succinate, maleate, fumarate, gluconate, saccharate, benzoate, methanesulfonate and pamoate [i.e., 1,1'-methylene-bis-(2-hydroxy-3-naphthoate)] salts. Although such salts must be pharmaceutically acceptable for administration to animals, it is often desirable in practice to initially isolate a compound of the formula I from the reaction mixture as a pharmaceutically unacceptable salt and then simply convert the latter back to the free base compound by treatment with an alkaline reagent, and subsequently convert the free base to a pharmaceutically acceptable acid addition salt. The acid addition salts of the base compounds of this invention are readily prepared by treating the base compound with a substantially equivalent amount of the chosen mineral or organic acid in an aqueous solvent medium or in a suitable organic solvent such as methanol or ethanol. Upon careful evaporation of the solvent, the desired solid salt is obtained.

Compounds of the formula that are acidic in nature are capable of forming base salts with various pharmacologically acceptable cations. These salts are all prepared by conventional techniques. The chemical bases that are used as reagents to prepare the pharmaceutically acceptable base salts of this invention are those which form non-toxic base salts with the acidic compounds of formula I. Such non-toxic base salts include those derived from such pharmacologically acceptable cations as sodium, potassium, calcium and magnesium, etc. These salts can easily be prepared by treating the corresponding acidic compounds with an aqueous solution containing the desired pharmacologically acceptable cations, and then evaporating the resulting solution to dryness, preferably under reduced pressure. Alternatively, they may also be prepared by mixing lower alkanolic solutions of the acidic compounds and the desired alkali metal alkoxide together, and then evaporating the resulting solution to dryness in the same manner as before. In either case, stoichiometric quantities of reagents are preferably employed in order to ensure completeness of reaction and maximum yields of the desired final product.

The compounds of the formula I and the pharmaceutically acceptable salts thereof (hereinafter, also referred to, collectively, as "the active compounds of the invention") are useful for the treatment of neurodegenerative, psychotropic and drug or alcohol induced deficits and are potent opioid receptor ligands. The active compounds of the invention may therefore be used in the treatment of disorders and conditions, such as those enumerated above, that can be treated by modulatiing binding to an opioid receptor.

The ability of the compounds of formula I to bind to the various opioid receptors and their functional activity at such receptors can be determined as described below.

A male Sprague-Dawley rat is euthanized, and its brain removed to ice. The forebrain is dissected and added to a centrifuge tube containing 35ml of ice-cold Tris HCI pH 7.4. The brain is homogenized using a polytron at 50% maximal speed, and the suspension spun in a Sorvall centrifuge at 48,000 X g for 10 minutes. The pellet is resuspended in Tris as above and spun again. The pellet is resuspended using Tris to a final concentration of 100mg/ml (original wet weight). The receptor binding reaction is initiated with the addition of 2.5mg of tissue to each well of a polypropylene 96-well plate containing 1.5nM [³H]-DAMGO and the appropriate competitor in a total volume of 2500µl. Non-specific binding is estimated using 1µM naltrexone. The plate is incubated with shaking for 90 minutes at 25°C. The plate is harvested using rapid filtration onto Betaplate Filtermat B filters using a Skatron Green Machine cell harvester. The filtermat is washed with approximately 5 ml of Tris HCI pH 7.4 buffer and air-dried overnight. The filtermat is then counted on a Wallac Betaplate Counter, 60 seconds per sample, and the data analyzed using Datafitter software.

The compositions of the present invention may be formulated in a conventional manner using one or more pharmaceutically acceptable carriers. Thus, the active compounds of the invention may be formulated for oral, buccal, transdermal (e.g., patch), intranasal, parenteral (e.g., intravenous, intramuscular or subcutaneous) or rectal administration or in a form suitable for administration by inhalation or insufflation.

For oral administration, the pharmaceutical compositions may take the form of, for example, tablets or capsules prepared by conventional means with pharmaceutically acceptable excipients such as binding agents (e.g., pregelatinised maize starch, polyvinylpyrrolidone or hydroxypropyl methylcellulose); fillers (e.g., lactose, microcrystalline cellulose or calcium phosphate); lubricants (e.g., magnesium stearate, talc or silica); disintegrants (e.g., potato starch or sodium starch glycollate); or wetting agents (e.g., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, methyl cellulose or hydrogenated edible fats); emulsifying agents (e.g., lecithin or acacia); non-aqueous vehicles (e.g., almond oil, oily esters or ethyl alcohol); and preservatives (e.g., methyl or propyl p-hydroxybenzoates or sorbic acid).

For buccal administration the composition may take the form of tablets or lozenges formulated in conventional manner.

The active compounds of the invention may be formulated for parenteral administration by injection, including using conventional catheterization techniques or infusion. Formulations for injection may be presented in unit dosage form, e.g., in ampules or in multi-dose containers, with an added preservative. The compositions may take such forms as suspensions, solutions or emulsions in oily or aqueous vehicles, and may contain formulating agents such as suspending, stabilizing and/or dispersing agents. Alternatively, the active ingredient may be in powder form for reconstitution with a suitable vehicle, e.g., sterile pyrogen-free water, before use.

The active compounds of the invention may also be formulated in rectal compositions such as suppositories or retention enemas, e.g., containing conventional suppository bases such as cocoa butter or other glycerides.

For intranasal administration or administration by inhalation, the active compounds of the invention are conveniently delivered in the form of a solution or suspension from a pump spray container that is squeezed or pumped by the patient or as an aerosol spray presentation from a pressurized container or a nebulizer, with the use of a suitable propellant, e.g., dichlorodifluoromethane, trichiorofluoromethane, dichlorotetrafluoroethane, carbon dioxide or other suitable gas. In the case of a pressurized aerosol, the dosage unit may be determined by providing a valve to deliver a metered amount. The pressurized container or nebulizer may contain a solution or suspension of the active compound. Capsules and cartridges (made, for example, from gelatin) for use in an inhaler or insufflator may be formulated containing a powder mix of a compound of the invention and a suitable powder base such as lactose or starch.

In general, a therapeutically effective daily oral or intravenous dose of the compounds of formula (I) and their salts is likely to range from 0.001 to 50 mg/kg body weight of the subject to be treated, preferably 0.1 to 20 mg/kg. The compounds of the formula (I) and their salts may also be administered by intravenous infusion, at a dose which is likely to range from 0.001-10 mg/kg/hr.

Tables or capsules of the compounds may be administered singly or two or more at a time as appropriate. It is also possible to administer the compounds in sustained release formulations.

The physician will determine the actual dosage which will be most suitable for an individual patient and it will vary with the age, weight and response of the particular patient. The above dosages are exemplary of the average case. There can, of course, be individual instances where higher or lower dosage ranges are merited, and such are within the scope of this invention.

Alternatively, the compounds of the formula (I) can be administered by inhalation or in the form of a suppository or pessary, or they may be applied topically in the form of a lotion, solution, cream, ointment or dusting powder. An alternative means of transdermal administration is by use of a skin patch. For example, they can be incorporated into a cream consisting of an aqueous emulsion of polyethylene glycols or liquid paraffin. They can also be incorporated, at a concentration of between 1 and 10% by weight, into an ointment consisting of a white wax or white soft paraffin base together with such stablisers and preservatives as may be required.

The following Examples illustrate the preparation of the compounds of the present invention. Commercial reagents were utilized without further purification. All NMR data were recorded at 250, 300 or 400 MHz in deuterochloroform unless otherwise specified and are reported in parts per million (δ) and are referenced to the deuterium lock signal from the sample solvent. All non-aqueous reactions were carried out in dry glassware with dry solvents under an inert atmosphere for convenience and to maximize yields. All reactions were stirred with a magnetic stirring bar unless otherwise stated. Unless otherwise stated, all mass spectra were obtained using chemical impact conditions. Ambient or room temperature refers to 20-25°C.

### PREPARATION A

### 4-(3-Methoxy-phenyl)-1,3,3-trimethyl-azetidin-2-one.

To a solution of (3-methoxy-benzylidene)-methyl-amine (3.0 g, 20 mmol) in methylene chloride (20 mL) at room temperature was added TiCl4 (IM in CH₂Cl₂, 20 n-tL) over 10 minutes. The reaction mixture was stirred at room temperature for 5 minutes. A solution of the (Imethoxy-vinyloxy)-trimethyl-silane in methylene chloride (5 mL) was added and the mixture was stirred at room temperature for 10 hours. The reaction mixture was then poured into 50 mL ice-water and the biphasic mixture was stirred vigorously for 30 minutes. The layers were separated and the aqueous layer was washed with methylene chloride (3x4O mL). The combined organic layers were dried (Na₂SO₄) and concentrated. Purification by flash chromatography with hexanes/EtOAc (1:1) afforded 3. 9 g (88% yield) of 4-(3-Methoxyphenyl)-1,3,3-trimethyl-azetidin-2-one. ¹HNMR (400 MHz, CDCl₃,) δ 7.28 (ap t, 1H), 6.84 (dd, J = 8.1, 2.1 Hz, 1H), 6.73 (d, J 7.5 Hz, 1H), 4.25 (s, 1H), 3.80 (s, 3H), 2.85 (s, 3H), 1.40 (s, 3H), 0.78 (s, 3H); MS (M+1) 220.2.

### PREPARATION B

### 3-(3-methoxy-phenyl)-3-methylaminopropionamides.

To a solution of the primary amine (5 equivalents) in tetrahydrofuran (0.5 M) at -78 C was added n-butyllithium (4.5 equivalents) dropwise over 2 minutes. The reaction mixture was stirred at -78 C for 5 minutes. A solution of the lactam (1 equivalent) in tetrahydrofuran (1M) was added dropwise and the mixture was stirred at - 78°C for 30° minutes and at 0°C for 2 hours. The reaction was quenced by addition of water and the aqueous layer was washed with ether. The organic layer was dried (Na₂SO₄) and concentrated. Purification by flash chromatography afforded the desired 3-(3-methoxy-phenyl)-3-methylamino-propionamides in yields ranging from 75% to 96%.

The title compounds of Preparations C-E were prepared by a method analogous to that described in Preparation B.

### PREPARATION C

### N-Benzyl-3-(3-methoxy-phenyl)-2,2-dimethyl-3-methylamino-propionamide

¹HNMR (400 MHz, CDCl₃) δ 6.82-6.72 (comp, 3 H), 4.46-4.40 (comp, 2H), 3.77 (s, 3H), 3.59 (s, 1H), 2.18 (s, 3H), 1.14 (s, 3H), 1.06 (s, 3H); MS (M+ 1) 327.2.

### PREPARATION D

### N-Cyclohexylmethyl-3-(3-methoxy-phenyl)-2,2-dimethyl-3-methylamino-propionamide

¹HNMR (400 MHz, CDCl₃) δ 7.54 (br, 1H), 7.23-7.18 (m, 1H), 6.82-6.75 (comp, 3H), 3.78 (s, 3H), 3.57 (s, 1H), 3.15-3.01 b(comp, 2H), 2.21 (s, 3H), 1.11 (s, 3H), 1.03 (s, 1H), 0.97-0.86 (comp, 1H), 0.49-0.43 (comp, 2H), 0.19-0.14 (comp, 2H); MS (M+1) 291.2.

### PREPARATION E

### N-Cyclopropylmethyl-3-(3-methoxy-phenyl)-2,2-dimethyl-3-methylaminopropionamide

¹HNMR (400 MHz, CDCl₃) δ 7.71 (br, 1H), 7.19 (t, 1H), 6.80-6.75 (comp, 3H), 3.76 (s, 3H), 3.55 (s, 1H), 3.09-2.95 (comp, 2H), 2.19 (s, 3H), 1.47-1.38 (m, 1H), 1.09 (s, 3H), 0.99 (s, 3H), 0.94-0.84 (comp, 2H); MS (M+1) 333.3.

### EXAMPLE 1

### 3-(3-hydroxy-phenyl)-3-methylaminopropionamides

The corresponding -3-(3-methoxy-phenyl)-3-methylamino-propionamide was dissolved in a solution of acetic acid and 48% aqueous hydrogen bromide (1:1, 0.5-0.3 M overall). The reaction mixture was heated to 110°C for 4-6 hours. The mixture was then cooled to room temperature and was basified to PH 9 with saturated aqueous ammonium hydroxide. The aqueous layer was washed with methylene chloride and the organic layer was dried (Na₂SO₄) and concentrated. Purification by flash chromatography afforded the desired -3-(3-hydroxyphenyl)-3-methylamino-propionamides in yields ranging from 60% to 85%.

The title compounds of Examples 2-11 were prepared by a method analogous to that described in Example 1.

### EXAMPLE 2

### 1-[(3-Hydroxy-phenyl)-methylamino-methyl]-cyclohexanecarboxylic acid benzylamide

¹NMR (400 MHz, CDCl₃) δ 7.11 (t, 1H), 6.93 (s, 1H), 6.84 (d, 1H), 6.56 (d, 1H), 6.48 (br, 1H) 4.58-4.30 (m, 1H), 4.20-3.75 (m, 1H), 3.81 (s, 1H), 2.25 (s, 3H); MS (M+1) 353.2.

### EXAMPLE 3

### 1-[(3-Hydroxy-phenyl)-methylamino-methyl]-cyclohexanecarboxylic acid (naphthalen-1-ylmethyl)-amide

¹HNMR (400 MHz, CDCl₃) δ 7.97 (d, 1H), 7.84 (d, 1H), 7.82 (d, 1H), 7.03 (t, 1H), 6.8 (br, 1H), 6.70 (dd, 1H), 4.89-4.79 (comp, 1H),3.38 (s, 1H); MS (M+1) 403.2.

### EXAMPLE 4

### 1-[(3-Hydroxy-phenyl)-methylamino-methyl]-cyclohexanecarboxylic acid (3-phenylpropyl)-amide

¹HNMR (400 MHz, CDCl₃) δ 6.83 (d, 1H), 6.79 (s, 1H), 6.63 (d, 1H), 3.35 (br, 1H), 3.49 (s, 1H), 3.36-3.30 (comp, 2H), 2.59 (t, 2H), 2.25 (s, 3H); MS (M+1) 381.2.

### EXAMPLE 5

### 1-[(3-Hydroxy-phenyl)-methylamino-methyl]-cyclohexanecarboxylic acid phenethylamide

¹HNMR (400 MHz, CDCl₃) δ 6.75 (d, 1H), 6.66 (s, 1H), 6.60 (d, 1H), 6.41 (br, 1H), 3.58-4.42 (comp, 2H), 3.38 (s, 1H), 2.85-2.69 (comp, 2H), 2.16 (s, 3H); MS (M+1) 367.3.

### EXAMPLE 6

### 3-(3-Hydroxy-phenyl)-2,2-dimethyl-3-methylamino-N-phenethyl-propionamide

¹HNMR (400 MHz, CDCl₃) δ 7.77 (br, 1H), 6.80-6.65 (comp, 2H), 6.62 (d, 1H), 2.81-2.73 (comp, 2H), 2.10 (s, 3H), 1.06 (s, 3H), 0.99 (s, 3H); MS (M+1) 327.3.

### EXAMPLE 7

### 3-(3-Hydroxy-phenyl)-2,2-dimethyl-3-methylamino-N-naphthalen-1-ylmethyl-propionamide

¹HNMR (400 MHz, CDCl₃) δ 8.32-8.30 (m, 1H), 7.94 (d, 1H), 7.82 (d, 1H), 6.74 (dd, 1H), 6.69 (s, 1H), 6.62 (d, 1H), 4.89-4.82 (comp, 2H), 3.50 (s, 1H), 2.03 (s, 3H), 1.09 (s, 3H), 1.06 (s, 3H); MS (M+1) 363.2.

### EXAMPLE 8

### 3-(3-Hydroxy-phenyl)-2,2-dimethyl-3-methylamino-N-(3-phenylpropyl)-propionamide

¹HNMR (400 MHz, CDCl₃) δ 7.73 (br, 1H), 6.78-6.74 (comp, 2H), 6.69 (d, 1H), 3.52 (s, 1H), 3.30-3.20 (comp, 2H), 2.61 (t, 2H), 2.20 (s, 3H), 1.86-1.78 (comp, 2H), 1.07 (s, 3H), 0.99 (s, 3H); MS (M+1) 341.3.

### EXAMPLE 9

### N-Cyclopropylmethyl-3-(3-hydroxy-phenyl)-2,2-dimethyl-3methylamino-propionamide

¹HNMR (400 MHz, CDCl₃) δ 7.58 (br, 1H), 7.14 (s, 1H), 6.81 (s, 1H), 6.77 (dd, 1H), 6.72 (d, 1H)3.57 (s, 1H), 3.19-3.04 (comp, 2H), 2.24 (s, 3H), 1.14 (s, 3H), 1.07 (s, 3H), 0.97-0.88 (m, 1H) 0.50-0.41 (comp, 2H), 0.18-0.14 (comp, 2H); MS (M+1)277.2.

### EXAMPLE 10

### N-Cyclohexylmethyl-3-(3-hydroxy-phenyl)-2.2-dimethyl-3methylamino-propionamide

¹HNMR (400 MHz, CDCl₃) δ 7.15 (t, 1H), 6.80-6.75 (comp, 2H), 6.27 (d, 1H), 3.59 (s, 1H), 3.16-3.02 (comp, 2H), 2.25 v(s, 3H), 1,14 (s, 3H), 1.06 (s, 3H), 0.95-0.84 (comp, 2H); MS (M+1) 319.3.

### EXAMPLE 11

### N-Benzyl-3-(3-hydroxy-phenyl)-2,2-dimethyl-3-methylamino-propionamide

¹HNMR (400 MHz, CDCl₃) δ 7.08 (t, 1H), 6.73-6.65 (comp, 3H), 4.35 (AB q, 2H), 3.67 (s, 1H), 2.12 (s, 3H), 1.14 (s, 3H), 1.04 (s, 3H); MS (M+ 1) 313.2.

### EXAMPLE 12

### 3-(3-hydroxy-phenyl)-3-methylamino-propionamides

To a solution of the -3-(3-hydroxy-phenyl)-3-methylamino-propionamide (1 equivalent) in methylene chloride (CH₂Cl₂) (0.4M) was added the aldehyde (1.2 equivalents) followed by addition of acetic acid (1.2 equivalents) and NaBH(OAc)₃ (1.5 equivalents). The reaction mixture was stirred at room temperature for 16 hours. The mixture was then partitioned between equal volumes of CH₂Cl₂ and sat. aqueous sodium bicarbonate (NaHCO₃). The organic layer was separated and the aqueous layer was washed with CH₂Cl₂ (3X). The combined organic layers were dried (MgSO₄) and concentrated. Purification by flash chromatography afforded the desired tertiary amines in yields ranging from 60-95%.

The title compounds of Examples 13-16 were prepared by a method analogous to that described in Example 12.

### EXAMPLE 13

### 3-(Cyclopropylmethyl-methyl-amino)-3-(3-hydroxy-phenyl)-2,2-dimethyl-N-phenethyl-propionamide

¹HNMR (400 MHz, CDCl₃) δ 8.54 (br, 1H), 7.09 (t, 1H), 6.76 (dd, 1H), 6.69 (s, 1H), 6.64 (d, 1H), 3.72-3.60 M, 1H), 3.54 (s, 1H), 2.39-2.01 (comp, 2H), 1.14 (s, 3H), 0.38-0.30 (comp, 2H); MS (M+1) 381.1.

### EXAMPLE 14

### 3-(3-Hydroxy-phenyl)-2,2-dimethyl-3-(methyl-thiazol-2-ylmethyl-amino)-N-phenethyl-propionamide

¹HNMR (400 MHz, CDCl₃) δ 7.84 (br, 1H), 7.66 (d, 1H), 6.84 (s, 1H), 6.80-6.74 (comp, 2H), 3.96 (d, 1H), 3.86 (s, 1H), 3.66-3.55 (m, 1H), 3.52 (d, 1H), 2.12 (s, 3H), 1.09 (s, 3H); MS (M+1) 424.1.

### EXAMPLE 15

### 3-(3-Hydroxy-phenyl)-2,2-dimethyl-3-(methyl-propyl-amino ) -N-phenethyl-propionamide

¹HNMR (400 MHz, CDCl₃) δ 8.92 (br, 1H), 7.09 (t, 1H), 6.82 (dd, 1H), 6.75 (s, 1H), 6.61 (d, 1H), 3.67-3.58 (m, 1H), 2.45-3.35 (m, 1H), 2.19-2.10 (m, 1H), 1.08 (s, 3H), 0.74 (t, 3H); MS (M+ 1) 369.1.

### EXAMPLE 16

### 3-(3-Hydroxy-phenyl)-3-(isobutyl-methyl-amino)-2,2-dimethyl-N-phenethyl-propionamide

¹HNMR (400 MHz, CDCl₃) δ 8.50-8.45 (m, 1H), 6.76 (dd, 1H), 6,69 (s, 1H), 6.65 (d, 1H), 3.66-3.60 (m, 1H), 3.50 (s, 1H), 2.47-3.38 (m, 1h), 1.40-1.30 (m, 1H), 1.09 (s. 3H); MS (M+ 1) 397.1.

## Claims

1. A compound of the formula 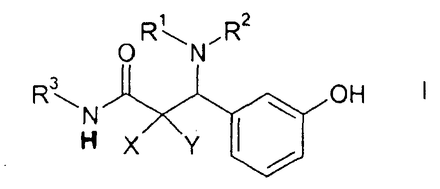 or the pharmaceutically acceptable salt thereof; wherein
R¹, R², and R³ are each independently selected from hydrogen, (C₁-C₆)alkyl, (C₅-C₁₀)aryl, (C₆-C₁₀)aryl(C₁-C₆)alkyl, (C₂-C₉)heteroaryl, (C₂-C₉)heteroaryl(C₁-C₅)alkyl, (C₃-C₇)cycloalkyl, (C₃-C₇)cycloalkyl(C₁-C₆)alkyl, (C₂-C₉)heterocycloalkyl and (C₂-C₉)heterocycloalkyl(C₁-C₆)alkyl wherein each alkyl, aryl, heteroaryl, cycloalkyl or heterocycloalkyl may be optionally substituted by one to four substituents selected from the group consisting of carboxy, cyano, amino, deuterium, hydroxy, (C₁-C₆)alkyl, (C₁-C₆)alkoxy, halo, (C₁-C₆)acyl, (C₁-C₆)alkylamino, amino(C₁-C₆)alkyl, (C₁-C₆)alkoxy-CO-NH, (C₁-C₆)alkylamino-CO-, (C₂-C₆)alkenyl, (C₂-C₆) alkynyl, (C₁-C₆)alkylamino, amino(C₁-C₆)alkyl, hydroxy(C₁-C₆)alkyl, (C₁-C₆)alkoxy(C₁-C₆)alkyl, (C₁-C₆)alkoxy(C₁-C₆)alkyl, nitro, cyano(C,-C₆)alkyl, halo(C₁-C₆)alkyl, nitro(C₁-C₆)alkyl, trifluoromethyl, trifluoromethyl(C₁-C₆)alkyl, (C₁-C₆)acylamino, (C₁-C₆)acylamino(C₁-C₆)alkyl, (C₁-C₆)alkoxy(C₁-C₆)acylamino, amino(C₁-C₆)acyl, amino(C₁-C₆)acyl(C₁-C₆)alkyl, (C₁-C₆)alkylamino(C₁-C₆)acyl, ((C₁-C₆)alkyl)₂amino(C₁-C₆)acyl, R⁴R⁵N-CO-O-, R⁴R⁵N-CO-(C₁-C₆)alkyl, (C₁-C₆)alkyl-S(O)ₘ, R⁴R⁵NS(O)ₘ, R⁴R⁵NS(O)ₘ (C₁-C₆)alkyl, R⁴S(O)ₘ R⁵N, R⁴S(O)ₘR⁵N(C₁-C₆)alkyl wherein m is 0, 1 or 2 and R⁴ and R⁵ are each independently selected from hydrogen or (C₁-C₆)alkyl; and
X and Y are each independently hydrogen or (C₁₋C₃)alkyl;
or X and Y may be taken together with the carbon to which they are attached to form a compound of formula II 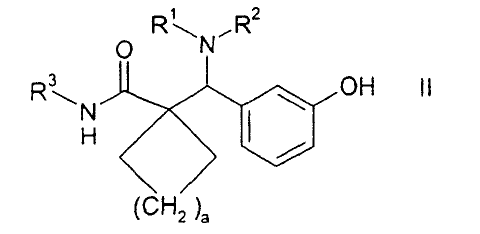 wherein a is 0, 1, 2, 3, or 4; and
R¹, R² and R³ are as defined above.

2. A compound according to claim 1, wherein X is hydrogen, (C₁-C₆)alkyl or (C₃-C₇)cycloalkyl.

3. A compound according to claim 1, wherein Y is hydrogen, (C₁-C₆)alkyl or (C₃-C₇)cycloalkyl.

4. A compound according to claim 1, wherein R¹ is hydrogen or (C₁-C₆)alkyl.

5. A compound according to claim 1, wherein R² is (C₁-C₆)alkyl, (C₃₋C₇)cycloalkyl(C₁-C₆)alkyl or trifluoromethyl.

6. A compound according to claim 1, wherein R³ is (C₃-C₇)cycloalkyl(C₁-C₇)alkyl or (C₆-C₁₀)aryl(C₁-C₆)alkyl.

7. A compound according to claim 1, wherein X is hydrogen, (C₁-C₆)alkyl or (C₃-C₇)cycloalkyl; Y is hydrogen, (C₁-C₆)alkyl or (C₃-C₇)cycloalkyl; R¹ is hydrogen or (C₁-C₆)alkyl; R² is (C₁-C₆)alkyl, (C₃-C₇)cloalkyl(C₁-C₆)alkyl or trifluoromethyl; and R³ is (C₃-C₇)cycloalky(C₁-C₇)alkyl or (C₆-C₁₀)aryl(C₁-C₆)alkyl.

8. A pharmaceutical composition for treating a disorder or condition selected from inflammatory diseases such as arthritis, psoriasis, asthma, or inflammatory bowel disease, disorders of respiratory function such as asthma, cough and apnea, allergies, gastrointestinal disorders such as gastritis, functional bowel disease, irritable bowel syndrome, functional diarrhoea, functional distension, functional pain, nonulcerogenic dyspepsia and other disorders of motility or secretion, and emesis, stroke, shock, brain edema, head trauma, spinal cord trauma, cerebral ischemia, cerebral deficits subsequent to cardiac bypass surgery and grafting, urogential tract disorders such as urinary incontinence, chemical dependencies and addictions (e.g., addictions to or dependencies on alcohol, opiates, benzodiazepines, nicotine, heroin or cocaine), chronic pain, nonsomatic pain, acute pain and neurogenic pain, systemic lupus erythematosis, analgesia, addiction, immunotherapy, appetite control, gastrointestinal dysfunction, Hodgkin's disease, Sjogren's disease, epilepsy and rejection in organ transplants and skin grafts in a mammal, comprising an amount of a compound according to claim 1 that is effective in treating such disorder or condition and a pharmaceutically acceptable carrier.

9. A pharmaceutical composition for treating a disorder or condition, the treatment or prevention of which can be effected or facilitated by modulating binding to mu opioid receptors in a mammal, comprising an amount of a compound according to claim 1 that is effective in treating such disorder or condition and a pharmaceutically acceptable carrier.

10. The use of an effective amount of a compound according to Claim 1 for the preparation of a medicament for the treatment of a disorder or condition selected from inflammatory diseases such as arthritis, psoriasis, asthma, or inflammatory bowel disease, disorders of respiratory function such as asthma, cough and apnea, allergies, gastrointestinal disorders such as gastritis, functional bowel disease, irritable bowel syndrome, functional diarrhoea, functional distension, functional pain, nonulcerogenic dyspepsia and other disorders; of motility or secretion, and emesis, stroke, shock, brain edema, head trauma, spinal cord' trauma, cerebral ischemia, cerebral deficits subsequent to cardiac bypass surgery and grafting, urogenital tract disorders such as urinary incontinence, chemical dependencies and addictions (e.g. addictions to or dependencies on alcohol, opiates, benzodiazepines, nicotine, heroin or cocaine), chronic pain, nonsomatic pain, acute pain and neurogenic pain, systemic lupus erythematosis, analgesia, addiction, immunotherapy, appetite control, gastrointestinal dysfunction, Hodgkin's disease, Sjogren's disease, epilepsy and rejection in organ transplants and skin grafts in a mammal.

11. The use of an effective amount of a compound according to Claim 1 for the preparation of a medicament for the treatment of a disorder of condition, the treatment of which, can be effected or facilitated by modulating binding to mu opioid receptors in a mammal.

12. A pharmaceutical composition for treating a disorder or condition selected from inflammatory diseases such as arthritis, psoriasis, asthma, or inflammatory bowel disease, disorders of respiratory function such as asthma, cough and apnea, allergies, gastrointestinal disorders such as gastritis, functional bowel disease, irritable bowel syndrome, functional diarrhoea, functional distension, functional pain, nonulcerogenic dyspepsia and other disorders of motility or secretion, and emesis, stroke, shock, brain edema, head trauma, spinal cord trauma, cerebral ischemia, cerebral deficits subsequent to cardiac bypass surgery and grafting, urogential tract disorders such as urinary incontinence, chemical dependencies and addictions (e.g., addictions to or dependencies on alcohol, opiates, benzodiazepines, nicotine, heroin or cocaine), chronic pain, nonsomatic pain, acute pain and neurogenic pain, systemic lupus erythematosis, analgesia, addiction, immunotherapy, appetite control, gastrointestinal dysfunction, Hodgkin's disease, Sjogren's disease, epilepsy and rejection in organ transplants and skin grafts in a mammal, comprising an opioid receptor binding modulating effective amount of a compound according to claim 1 and a pharmaceutically acceptable carrier.

13. A pharmaceutical composition for treating a disorder or condition, the treatment or prevention of which can be effected or facilitated by modulating binding to opioid receptors in a mammal, comprising an mu opioid receptor binding modulating effective amount of a compound according to claim 1 and a pharmaceutically acceptable carrier.

14. The use of an amount of a compound according to Claim 1 effective in modulating opioid receptor binding for the preparation of a medicament for the treatment of a disorder or condition selected from inflammatory diseases such as arthritis, psoriasis, asthma, or inflammatory bowel disease, disorders of respiratory function such as asthma, cough and apnea, allergies, gastrointestinal disorders such as gastritis, functional bowel disease, irritable bowel syndrome, functional diarrhoea, functional distension, functional pain, nonulcerogenic dyspepsia and other disorders of motility or secretion, and emesis, stroke, shock, brain edema, head trauma, spinal cord trauma, cerebral ischemia, cerebral deficits subsequent to cardiac bypass surgery and grating, urogenital tract disorders such as urinary incontinence, chemical dependencies and addictions (e.g. addictions to or dependencies on alcohol, opiates, benzodiazepines, nicotine, heroin or cocaine), chronic pain, nonsomatic pain, acute pain and neurogenic pain, systemic lupus erythematosis, analgesia, addiction, immunotherapy, appetite control, gastrointestinal dysfunction, Hodgkin's disease, Sjogren's disease, epilepsy and rejection in organ transplants and skin grafts in a mammal.

15. The use of an amount of a compound according to Claim 1 effective in modulating opioid receptor binding for the preparation of a medicament for the treatment or prevention of a disorder or condition which can be effected or facilitated by modulating binding to mu opiod receptors in a mammal.

## Patentansprüche

1. Verbindung der Formel 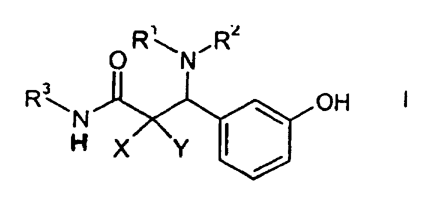 oder das pharmazeutisch akzeptable Salz derselben;
wobei
R¹' R²' und R³ unabhängig voneinander unter Wasserstoff, (C₁₋C₆)-Alkyl, (C₆₋C₁₀)-Aryl, (C₆₋C₁₀)-Aryl(C₁₋C₆)-alkyl, (C₂₋C₉)-Heteroaryl, (C₂₋C₉)-Heteroaryl (C₁₋C₅)-alkyl, (C₃₋C₇)-Cycloalkyl, (C₃₋C₇)-Cycloalkyl (C₁₋C₆)-alkyl, (C₂₋C₉)-Heterocycloalkyl und (C₂₋C₉)-Heterocycloalkyl(C₁₋C₆)-alkyl ausgewählt werden, wobei jedes Alkyl, Aryl, Heteroaryl, Cycloalkyl oder Heterocycloalkyl gegebenenfalls durch einen bis vier Substituenten substituiert sein kann, der bzw. die aus de Gruppe ausgewählt wird bzw. werden, die aus Carboxy, Cyano, Amino, Deuterium, Hydroxy, (C₁₋C₆)-Alkyl, (C₁₋C₆)-Alkoxy, Halo, (C₁₋C₆)-Acyl, (C₁₋C₆)-Alkylamino, Amino(C₁₋C₆)-alkyl, (C₁₋C₆)-Alkoxy-CO-NH, (C₁₋C₆)-Alkylamino-CO-, (C₂₋C₆)-Alkenyl, (C₂₋C₆)-Alkynyl, (C₁₋C₆)-Alkylamino, Amino(C₁₋C₆)-alkyl, Hydroxy(C₁₋C₆)-alkyl, (C₁₋C₆)-Alkoxy(C₁₋C₆)-alkyl, (C₁₋C₆)-Acyloxy(C₁₋C₆)-alkyl, Nitro, Cyano(C₁₋C₆)-alkyl, Halo(C₁₋C₆)-alkyl, Nitro(C₁₋C₆)-alkyl, Trifluormethyl, Trifluormethyl(C₁₋C₆)-alkyl, (C₁₋C₆)-Acylamino, (C₁₋C₆)-Acylamino (C₁₋C₆)-alkyl, (C₁₋C₆)-Alkoxy(C₁₋C₆)-acylamino, Amino(C₁₋C₆)-acyl, Amino(C₁₋C₆)-acyl(C₁₋C₆)-alkyl, (C₁₋C₆)-Alkylamino(C₁₋C₆)-acyl, ((C₁₋C₆)-Alkyl)₂-amino(C₁-C₆)-acyl, R⁴R⁵N-CO-O-, R⁴R⁵N-CO-(C₁₋C₆)-Alkyl, (C₁-C₆)-Alkyl-S(O)ₘ, R⁴R⁵NS(O)ₘ, R⁴R⁵NS(O)ₘ, (C₁₋C₆)-Alkyl, R⁴S(O)ₘR⁵N, R⁴S(O)ₘR⁵N (C₁₋C₆)-Alkyl besteht, wobei m 0, 1 oder 2 beträgt und R⁴ und R⁵ jeweils unabhängig voneinander unter Wasserstoff oder (C₁-C₅)-Alkyl ausgewählt werden; und
X und Y unabhängig voneinander für Wasserstoff oder (C₁₋C₃)-Alkyl stehen;
oder X und Y mit dem Kohlenstoff, an den sie angelagert sind, zusammengenommen werden können unter Bildung einer Verbindung der Formel II 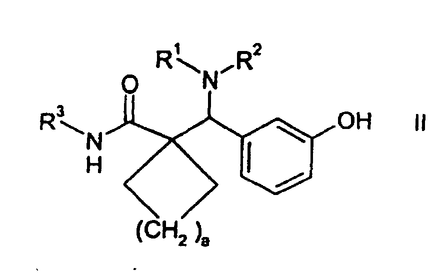 wobei a 0, 1, 2, 3 oder 4 beträgt; und
R¹, R² und R³ die oben angegebenen Definition aufweisen.

2. Verbindung nach Anspruch 1, wobei X für Wasserstoff, (C₁₋C₆)-Alkyl oder (C₃₋C₇)-Cycloalkyl steht.

3. Verbindung nach Anspruch 1, wobei Y für Wasserstoff, (C₁₋C₆)-Alkyl oder (C₃₋C₇)-Cycloalkyl steht.

4. Verbindung nach Anspruch 1, wobei R¹ für Wasserstoff oder (C₁-C₆)-Alkyl steht.

5. Verbindung nach Anspruch 1, wobei R¹ für (C₁-C₆)-Alkyl, (C₃-C₇)-Cycloalkyl (C₁-C₆)-alkyl oder Trifluormethyl steht.

6. Verbindung nach Anspruch 1, wobei R³ für (C₃-C₇)-Cycloalkyl(C₁₋C₇)-alkyl oder (C₆₋C₁₀)-Aryl(C₁₋C₆)-alkyl steht.

7. Verbindung nach Anspruch 1, wobei X für Wasserstoff, (C₁₋C₆)-Alkyl oder (C₃₋C₇)-Cycloalkyl, Y für Wasserstoff, (C₁₋C₆)-Alkyl oder (C₃₋C₇)-Cycloalkyl, R¹ für Wasserstoff oder (C₁₋C₆)-Alkyl, R² für (C₁₋C₆)-Alkyl, (C₃₋C₇)-Cycloalkyl(C₁₋C₆)-alkyl oder Trifluormethyl und R³ für (C₃₋C₇)-Cycloalkyl(C₁₋C₇)-alkyl oder (C₆₋C₁₀)-Aryl(C₁₋C₆)-alkyl steht.

8. Pharmazeutische Zusammensetzung für die Behandlung einer krankhaften Störung oder eines krankhaften Zustands, ausgewählt unter entzündlichen Erkrankungen wie Arthritis, Psoriasis, Asthma, oder entzündlicher Darmerkrankung, Störungen der Atmungsfunktion wie Asthma, Husten und Apnoe, Allergien, Magen-Darm-Beschwerden wie Gastritis, funktioneller Darmerkrankung, irritablem spastischem Kolon, funktioneller Diarrhö, funktioneller Blähung, funktionellen Schmerzen, nicht zu Geschwüren führender Dyspepsie und anderen Motilitäts- oder Sekretionsstörungen und Emesis, Schlaganfall, Schock, Hirnödem, Kopftrauma, Rückenmarkverletzung, Hirnischämie, Zerebraldefizit auf eine Herz-Bypass-Operation und Transplantation hin, Urogenitaltraktstörungen wie Harninkontinenz, Drogenabhängigkeit und -süchtigkeit (z.B. Alkohol-, Opiat-, Benzodiazepin-, Nikotin-, Heroin- oder Kokainabhängigkeit bzw. -süchtigkeit), chronischen Schmerzen, nichtsomatischen Schmerzen, akuten Schmerzen und neurogenen Schmerzen, systemischem Lupus erythematodes, Analgesie, Süchtigkeit, Immunotherapie, Appetitzüglung, Verdauungsbeschwerden, Hodgkin-Krankheit, Sjögren-Syndrom, Epilepsie und Abstoßung bei Organtransplantationen und Hauttransplantionen bei einem Säuger, umfassend eine Menge einer Verbindung nach Anspruch 1, die bei der Behandlung einer derartigen krankhaften Störung oder einem derartigen krankhaften Zustand wirksam ist, und einen pharmazeutisch akzeptablen Träger.

9. Pharmazeutische Zusammensetzung für die Behandlung einer krankhaften Störung oder eines krankhaften Zustands, deren bzw. dessen Behandlung oder Verhinderung durch Regulieren des Bindens an µ-opioide Rezeptoren bei einem Säuger durchgeführt oder erleichtert werden kann, umfassend eine Menge einer Verbindung nach Anspruch 1, die bei der Behandlung einer derartigen krankhaften Störung oder eines derartigen krankhaften Zustands wirksam ist, und einen pharmazeutisch akzeptablen Träger.

10. Verwendung einer wirksamen Menge einer Verbindung nach Anspruch 1 für die Zubereitung eines Medikaments für die Behandlung einer Beschwerde oder eines krankhaften Zustands, die bzw. der unter entzündlichen Erkrankungen wie Arthritis, Psoriasis, Asthma, oder entzündlicher Darmerkrankung, Störungen der Atmungsfunktion wie Asthma, Husten und Apnoe, Allergien, Magen-Darm-Beschwerden wie Gastritis, funktioneller Darmerkrankung, irritablem spastischem Kolon, funktioneller Diarrhö, funktioneller Blähung, funktionellen Schmerzen, nicht zu Geschwüren führender Dyspepsie und anderen Motilitäts- oder Sekretionsstörungen und Emesis, Schlaganfall, Schock, Hirnödem, Kopftrauma, Rückenmarkverletzung, Hirnischämie, Zerebraldefizit auf eine Herz-Bypass-Operation und Transplantation hin, Urogenitaltraktstörungen wie Harninkontinenz, Drogenabhängigkeit und -süchtigkeit (z.B. Alkohol-, Opiat-, Benzodiazepin-, Nikotin-, Heroin- oder Kokainabhängigkeit bzw. -süchtigkeit), chronischen Schmerzen, nichtsomatischen Schmerzen, akuten Schmerzen und neurogenen Schmerzen, systemischem Lupus erythematodes, Analgesie, Süchtigkeit, Immunotherapie, Appetitzüglung, Verdauungsbeschwerden, Hodgkin-Krankheit, Sjögren-Syndrom, Epilepsie und Abstoßung bei Organtransplantationen und Hauttransplantionen bei einem Säuger ausgewählt wird.

11. Verwendung einer wirksamen Menge einer Verbindung nach Anspruch 1 für die Zubereitung eines Medikaments für die Behandlung einer krankhaften Störung oder eines krankhaften Zustands, deren bzw. dessen Behandlung durch Regulieren des Bindens an µopioide Rezeptoren bei einem Säuger durchgeführt oder erleichtert werden kann,

12. Pharmazeutische Zusammensetzung für die Behandlung einer krankhaften Störung oder eines krankhaften Zustandes, die bzw. der unter entzündlichen Erkrankungen wie Arthritis, Psoriasis, Asthma, oder entzündlicher Darmerkrankung, Störungen der Atmungsfunktion wie Asthma, Husten und Apnoe, Allergien, Magen-Darm-Beschwerden wie Gastritis, funktioneller Darmerkrankung, irritablem spastischem Kolon, funktioneller Diarrhö, funktioneller Blähung, funktionellen Schmerzen, nicht zu Geschwüren führender Dyspepsie und anderen Motilitäts- oder Sekretionsstörungen und Emesis, Schlaganfall, Schock, Hirnödem, Kopftrauma, Rückenmarkverletzung, Hirnischämie, Zerebraldefizit auf eine Herz-Bypass-Operation und Transplantation hin, Urogenitaltraktstörungen wie Harninkontinenz, Drogenabhängigkeit und -süchtigkeit (z.B. Alkohol-, Opiat-, Benzodiazepin-, Nikotin-, Heroin- oder Kokainabhängigkeit bzw. -süchtigkeit), chronischen Schmerzen, nichtsomatischen Schmerzen, akuten Schmerzen und neurogenen Schmerzen, systemischem Lupus erythematodes, Analgesie, Süchtigkeit, Immunotherapie, Appetitzüglung, Verdauungsbeschwerden, Hodgkin-Krankheit, Sjögren-Syndrom, Epilepsie und Abstoßung bei Organtransplantationen und Hauttransplantionen bei einem Säuger ausgewählt wird, umfassend eine für das Regulieren des Bindens an einen opioiden Rezeptor wirksame Menge einer Verbindung nach Anspruch 1 und einen pharmazeutisch akzeptablen Träger.

13. Pharmazeutische Zusammensetzung für die Behandlung einer krankhaften Störung oder eines krankhaften Zustands, deren bzw. dessen Behandlung oder Verhinderung durch Regulieren des Bindens an opioide Rezeptoren bei einem Säuger durchgeführt oder erleichtert werden kann, umfassend eine für das Regulieren des Bindens an einen opioiden Rezeptor wirksame Menge einer Verbindung nach Anspruch 1 und einen pharmazeutisch akzeptablen Träger.

14. Verwendung einer Menge einer Verbindung nach Anspruch 1, die für das Regulieren des Bindens an einen opioiden Rezeptor wirksam ist, für die Zubereitung eines Medikaments für die Behandlung einer krankhaften Störung oder eines krankhaften Zustands, der bzw. die unter entzündlichen Erkrankungen wie Arthritis, Psoriasis, Asthma, oder entzündlicher Darmerkrankung, Störungen der Atmungsfunktion wie Asthma, Husten und Apnoe, Allergien, Magen-Darm-Beschwerden wie Gastritis, funktioneller Darmerkrankung, irritablem spastischem Kolon, funktioneller Diarrhö, funktioneller Blähung, funktionellen Schmerzen, nicht zu Geschwüren führender Dyspepsie und anderen Motilitäts- oder Sekretionsstörungen und Emesis, Schlaganfall, Schock, Hirnödem, Kopftrauma, Rückenmarkverletzung, Hirnischämie, Zerebraldefizit auf eine Herz-Bypass-Operation und Transplantation hin, Urogenitaltraktstörungen wie Harninkontinenz, Drogenabhängigkeit und -süchtigkeit (z.B. Alkohol-, Opiat-, Benzodiazepin-, Nikotin-, Heroin- oder Kokainabhängigkeit bzw. -süchtigkeit), chronischen Schmerzen, nichtsomatischen Schmerzen, akuten Schmerzen und neurogenen Schmerzen, systemischem Lupus erythematodes, Analgesie, Süchtigkeit, Immunotherapie, Appetitzüglung, Verdauungsbeschwerden, Hodgkin-Krankheit, Sjögren-Syndrom, Epilepsie und Abstoßung bei Organtransplantationen und Hauttransplantionen bei einem Säuger ausgewählt wird.

15. Verwendung einer Menge einer Verbindung nach Anspruch 1, die für das Regulieren des Bindens an einen opioiden Rezeptor wirksam ist, für die Zubereitung eines Medikaments für die Behandlung oder Verhinderung einer krankhaften Störung oder eines krankhaften Zustands, die durch Regulieren des Bindens an µ-opioide Rezeptoren bei einem Säuger durchgeführt oder erleichtert werden kann.

## Revendications

1. Composé de la formule 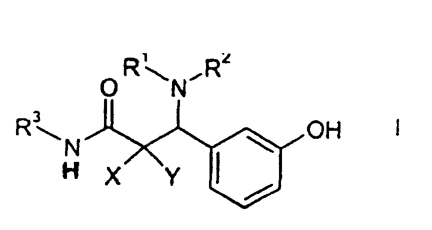 ou son sel acceptable du point de vue pharmaceutique; dans lequel
R¹' R²' et R³ sont chacun indépendamment sélectionnés parmi l'hydrogène, les résidus (C₁₋C₆)-alkyle, (C₆₋C₁₀)aryle, (C₆₋C₁₀)aryl(C₁₋C₆)alkyle, (C₂₋C₉)-hétéroaryle, (C₂₋C₉)hétéroaryl (C₁₋C₅) alkyle, (C₃-C₇)cycloalkyle, (C₃₋C₇)cycloalkyl(C₁₋C₆)alkyle, (C₂-C₉)hétérocycloalkyle et (C₂-C₉)hétérocycloalkyl(C₁-C₆)alkyle, dans lesquels chaque résidu alkyle, aryle, hétéroaryle, cycloalkyle ou hétérocycloalkyle peut être substitué en option par un ou plusieurs substituants sélectionnés parmi le groupe constitué des résidus carboxy, cyano, amino, deutérium, hydroxy, (C₁₋C₆)alkyle, (C₁₋C₆)alkoxy, halo, (C₁₋C₆)-acyle, (C₁₋C₆)alkylamino, amino(C₁₋C₆)alkyle, (C₁₋C₆)-alkoxy-CO-NH, (C₁₋C₆) alkylamino-CO-, (C₂₋C₆)-alkényle, (C₂₋C₆) alkynyle, (C₁₋C₆)alkylamino, amino(C₁₋C₆)alkyle, hydroxy(C₁₋C₆)alkyle, (C₁₋C₆)-alkoxy (C₁₋C₆)alkyle, (C₁₋C₆)acyloxy (C₁-C₆)alkyle, nitro, cyano(C₁₋C₆)alkyle, halo(C₁₋C₆)alkyle, nitro(C₁₋C₆)alkyle, trifluorométhyle, trifluorométhyl(C₁₋ C₆)alkyle, (C₁₋C₆)acylamino, (C₁₋C₆)acylamino(C₁₋C₆) alkyle, (C₁₋C₆)alkoxy(C₁₋C₆)acylamino, amino(C₁₋C₆)acyle, amino(C₁₋C₆)acyl(C₁₋C₆)alkyle, (C₁alkylamino(C₁₋C₆)acyle, ((C₁-C₆)alkyl)₂amino(C₁₋C₆)acyle, R⁴R⁵N-CO-O-, R⁴R⁵N-CO-(C₁₋C₆)alkyle, (C₁₋C₆)alkyl-S(O)ₘ, R⁴R⁵NS(O)ₘ, R⁴R⁵NS(O)ₘ, (C₁₋C₆) alkyle, R⁴S(O)ₘR⁵N, R⁴S(O)ₘR⁵N(C₁₋C₆)alkyle, dans lesquels m est 0, 1 ou 2 et R⁴ et R⁵ sont chacun indépendamment sélectionnés parmi l'hydrogène ou un résidu (C₁₋C₅)alkyle; et
X et Y sont chacun indépendamment l'hydrogène ou un résidu (C₁₋C₃)alkyle;
ou X et Y peuvent être pris ensemble avec le carbone auxquels ils sont attachés pour former un composé de formule II 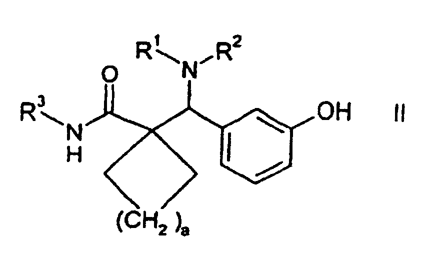 dans lequel a est 0, 1, 2, 3, ou 4; et
R¹, R² et R³ sont comme définis ci-dessus.

2. Composé selon la revendication 1, dans lequel X est l'hydrogène, un résidu (C₁₋C₆)alkyle ou (C₃₋C₇)cycloalkyle.

3. Composé selon la revendication 1, dans lequel Y est l'hydrogène, un résidu (C₁₋C₆)alkyle ou (C₃₋C₇)cycloalkyle.

4. Composé selon la revendication 1, dans lequel R¹ est l'hydrogène ou un résidu (C₁₋C₆)alkyle.

5. Composé selon la revendication 1, dans lequel R² est un résidu (C₁₋C₆)alkyle, (C₃₋C₇)cycloalkyl(C₁₋C₆)alkyle ou trifluorométhyle.

6. Composé selon la revendication 1, dans lequel R³ est un résidu (C₃₋C₇)cycloalkyl(C₁₋C₇)alkyle ou (C₆-C₁₀)aryl (C₁₋C₆)alkyle.

7. Composé selon la revendication 1, dans lequel X est l'hydrogène, un résidu (C₁₋C₆)alkyle ou (C₃₋C₇)cycloalkyle ; Y est l'hydrogène, un résidu (C₁₋C₆)alkyle ou (C₃₋C₇)cycloalkyle; R¹ est l'hydrogène ou un résidu (C₁-C₆)alkyle ; R² est un résidu (C₁₋C₆)alkyle, (C₃₋C₇)cycloalkyl(C₁₋C₆)alkyle ou trifluorométhyle; et R³ est un résidu (C₃₋C₇)cycloalkyl(C₁₋C₇)alkyle ou (C₆₋C₁₀)aryl (C₁₋C₆)alkyle.

8. Composition pharmaceutique en vue du traitement d'un désordre ou d'une maladie sélectionné(e) parmi les maladies inflammatoires, comme l'arthrite, le psoriasis, l'asthme, ou la maladie entérique inflammatoire, les désordres de la fonction respiratoire, comme l'asthme, la toux et l'apnée, des allergies, des désordres gastro-intestinaux, comme la gastrite, la maladie entérique fonctionnelle, le syndrome du côlon irritable, la diarrhée fonctionnelle, la distension fonctionnelle, la douleur fonctionnelle, la dyspepsie non ulcérogénique et d'autres désordres de la motilité ou de la sécrétion, et le vomissement, l'apoplexie, le choc, l'oedème cérébral, les traumatismes de la tête, les traumatismes de la moelle épinière, l'ischémie cérébrale, les déficits cérébraux consécutifs à une chirurgie de prothèse artérielle et de greffe, les désordres du tractus urogénital, comme l'incontinence urinaire, les dépendances chimiques et les dépendances, par exemple, vis-à-vis de l'alcool, des opiacés, des benzodiazépines, de la nicotine, de l'héroïne ou de la cocaïne ; les douleurs chroniques, les douleurs non somatiques, les douleurs aiguës, les douleurs neurogéniques, le lupus érythémateux disséminé, l'analgésie, la dépendance, l'immunothérapie, le contrôle de l'appétit, la dysfonction gastro-intestinale, la maladie de Hodgkin, la maladie de Sjogren, l'épilepsie et les phénomènes de rejet dans les transplantations d'organe et les greffes cutanées chez un mammifère, comprenant une quantité d'un composé selon la revendication 1, qui est efficace dans le traitement d'un désordre ou d'une maladie de ce genre et un excipient acceptable du point de vue pharmaceutique.

9. Composition pharmaceutique en vue du traitement d'un désordre ou d'une maladie, dont le traitement ou la prévention peut être effectué(e) ou facilité(e) par modulation de la liaison aux récepteurs opioïdes mu chez un mammifère, comprenant une quantité d'un composé selon la revendication 1 qui est efficace en vue du traitement d'un désordre ou d'une maladie de ce genre et un excipent acceptable du point de vue pharmaceutique.

10. Utilisation d'une quantité efficace d'un composé selon la revendication 1, pour la préparation d'un médicament pour le traitement d'un désordre ou d'une maladie sélectionné(e) parmi les maladies inflammatoires comme l'arthrite, le psoriasis, l'asthme, ou la maladie entérique inflammatoire, les désordres de la fonction respiratoire comme l'asthme, la toux et l'apnée, des allergies, des désordres gastro-intestinaux comme la gastrite, la maladie entérique fonctionnelle, le syndrome du côlon irritable, la diarrhée fonctionnelle, la distension fonctionnelle, la douleur fonctionnelle, la dyspepsie non ulcérogénique et d'autres désordres de la motilité ou de la sécrétion, et le vomissement, l'apoplexie, le choc, l'oedème cérébral, les traumatismes de la tête, les traumatismes de la moelle épinière, l'ischémie cérébrale, les déficits cérébraux consécutifs à une chirurgie de prothèse artérielle et de greffe, les désordres du tractus urogénital comme l'incontinence urinaire, les dépendances chimiques et les dépendances, par exemple, vis-à-vis de l'alcool, des opiacés, des benzodiazépines, de la nicotine, de l'héroïne ou de la cocaïne ; les douleurs chroniques, les douleurs non somatiques, les douleurs aiguës, les douleurs neurogéniques, le lupus érythémateux disséminé, l'analgésie, la dépendance, l'immunothérapie, le contrôle de l'appétit, la dysfonction gastro-intestinale, la maladie de Hodgkin, la maladie de Sjogren, l'épilepsie et les phénomènes de rejet dans les transplantations d'organe et les greffes cutanées chez un mammifère.

11. Utilisation d'une quantité efficace d'un composé selon la revendication 1 en vue de la préparation d'un médicament pour le traitement d'un désordre ou d'une maladie, dont le traitement peut être effectué ou facilité par une liaison modulante aux récepteurs opioïdes mu chez un mammifère.

12. Composition pharmaceutique en vue du traitement d'un désordre ou d'une maladie sélectionné(e) par les maladies inflammatoires comme l'arthrite, le psoriasis, l'asthme, ou la maladie entérique inflammatoire, les désordres de la fonction respiratoire comme l'asthme, la toux et l'apnée, des allergies, des désordres gastro-intestinaux, comme la gastrite, la maladie entérique fonctionnelle, le syndrome du côlon irritable, la diarrhée fonctionnelle, la distension fonctionnelle, la douleur fonctionnelle, la dyspepsie non ulcérogénique et d'autres désordres de la motilité ou de la sécrétion, et le vomissement, l'apoplexie, le choc, l'oedème cérébral, les traumatismes de la tête, les traumatismes de la moelle épinière, l'ischémie cérébrale, les déficits cérébraux consécutifs à une chirurgie de prothèse artérielle et de greffe, les désordres du tractus urogénital, comme l'incontinence urinaire, les dépendances chimiques et les addictions (par exemple, les addictions ou les dépendances vis-à-vis de l'alcool, des opiacés, des benzodiazépines, de la nicotine, de l'héroïne ou de la cocaïne), les douleurs chroniques, les douleurs non somatiques, les douleurs aiguës, les douleurs neurogéniques, le lupus érythémateux disséminé, l'analgésie, l'addiction, l'immunothérapie, le contrôle de l'appétit, la dysfonction gastro-intestinale, la maladie de Hodgkin, la maladie de Sjogren, l'épilepsie et les phénomènes de rejet dans les transplantations d'organe et les greffes cutanées chez un mammifère, comprenant une quantité efficace, modulant la liaison aux récepteurs opioïdes, d'un composé selon la revendication 1, et un excipient acceptable du point de vue pharmaceutique.

13. Composition pharmaceutique en vue du traitement d'un désordre ou d'une maladie, dont le traitement ou la prévention peut être effectué(e) ou facilité(e) par une liaison modulante aux récepteurs opioïdes mu chez un mammifère, comprenant une quantité efficace, modulant la liaison aux récepteurs opioïdes mu, d'un composé selon la revendication 1 et un excipent acceptable du point de vue pharmaceutique.

14. Utilisation d'une quantité d'un composé selon la revendication 1, effective dans la modulation de la liaison aux récepteurs opioïdes, en vue de la préparation d'un médicament pour le traitement d'un désordre ou d'une maladie sélectionné(e) parmi les maladies inflammatoires comme l'arthrite, le psoriasis, l'asthme, ou la maladie entérique inflammatoire, les désordres de la fonction respiratoire comme l'asthme, la toux et l'apnée, des allergies, des désordres gastro-intestinaux, comme la gastrite, la maladie entérique fonctionnelle, le syndrome du côlon irritable, la diarrhée fonctionnelle, la distension fonctionnelle, la douleur fonctionnelle, la dyspepsie non ulcérogénique et d'autres désordres de la motilité ou de la sécrétion, et le vomissement, l'apoplexie, le choc, l'oedème cérébral, les traumatismes de la tête, les traumatismes de la moelle épinière, l'ischémie cérébrale, les déficits cérébraux consécutifs à une chirurgie de prothèse artérielle et de greffe, les désordres du tractus urogénital, comme l'incontinence urinaire, les dépendances chimiques et les dépendances, par exemple, vis-à-vis de l'alcool, des opiacés, des benzodiazépines, de la nicotine, de l'héroïne ou de la cocaïne ; les douleurs chroniques, les douleurs non somatiques, les douleurs aiguës, les douleurs neurogéniques, le lupus érythémateux disséminé, l'analgésie, la dépendance, l'immunothérapie, le contrôle de l'appétit, la dysfonction gastro-intestinale, la maladie de Hodgkin, la maladie de Sjogren, l'épilepsie et les phénomènes de rejet dans les transplantations d'organe et les greffes cutanées chez un mammifère.

15. Utilisation d'une quantité efficace d'un composé selon la revendication 1, efficace dans la modulation de la liaison aux récepteurs opioïdes en vue de la préparation d'un médicament pour le traitement ou la prévention d'un désordre ou d'une maladie, qui peut être effectué(e) ou facilité(e) par modulation de la liaison aux récepteurs opioïdes mu chez un mammifère.
